# EUROPÄISCHE PATENTANMELDUNG

(11) **EP 1 985 281 A1**
(43) Veröffentlichungstag der Anmeldung: **29.10.2008**
(21) Anmeldenummer: 07008476.9
(22) Anmeldetag: 26.04.2007
(51) Int. Cl.: A61K 8/37, C11C 3/04

(54) **Ester von Hexyldecanol mit kurzkettigen Fettsäuren**

(71) Anmelder: Cognis IP Management GmbH, 40589 Düsseldorf (DE)
(72) Erfinder: Ansmann, Achim Dr., 40699 Erkrath (DE); Dierker, Markus Dr., 40597 Düsseldorf (DE)

(57) **Zusammenfassung**

Die Erfindung betrifft Ester von Hexyldodecanol mit kurzkettigen Fettsäuren und ihre Verwendung in kosmetischen und/oder pharmazeutischen Zubereitungen.

## Beschreibung

### Gebiet der Erfindung

Die Erfindung betrifft Ester von Hexyldodecanol mit kurzkettigen Fettsäuren und ihre Verwendung in kosmetischen und/oder pharmazeutischen Zubereitungen.

### Stand der Technik

Im Bereich kosmetischer Zubereitungen für die Haut- und Haarpflege sowie im Bereich pharmazeutischer Zubereitungen werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper entwickelt und getestet.

Ester von Hexyldecanol mit Fettsäuren sind beispielsweise aus US 6,391,287 (L Oréal) bekannt, dort werden Hexyldecyl carpylate (=Octanoate), Hexyldecyl laurate (=Dodecanoate), Hexyldecyl palmitat (=Hexadecanoate) und Hexyldecyl stearate (0Octadecanoate) als Co-solubilisatoren für bizyklische aromatische Verbindungen beschrieben.

Weiterhin wird die Verbindung Hexyldecyl 2-ethylhexanoate von der Fa. Kokyu Alcohol Co, Ltd unter dem Handelsnamen ICEH vertrieben.

Es besteht allerdings weiterhin der Bedarf nach neuen Ölkörpem, die über ein flexibleres Einsatzspektrum verfügen (z.B. Kompatibilität mit weiteren kosmetischen Inhaltsstoffen) sowie den hohen Anforderungen an die Sensorik genügen. Von besonderem Interesse sind Ölkörper, welche auf der Haut ein als "leicht" bezeichnetes Gefühl erzeugen. Von großer Bedeutung sind die Verteilbarkeit und Spreitfähigkeit auf der Haut. Der moderne Verbraucher fordert von Ölkörper und den daraus hergestellten Zubereitungen, dass sie ein weiches Hautgefühl erzeugen und dass sich die Haut gepflegt anfühlt. Eine weitere Anforderung an moderne Rohstoffe für kosmetische und/oder pharmazeutische Zubereitungen ist, dass sie auf Basis nachwachsender, pflanzlicher Rohstoffe gewonnen werden können.

Aufgabe der vorliegenden Erfindung war es neue Stoffe bereit zu stellen, welche sich für kosmetischen und/oder pharmazeutischen Zubereitungen, insbesondere als Ölkörper/Emollients eignen und ein sensorisch besonders "leichtes" Hautgefühl erzeugen. Weiterhin sollten diese Stoffe auf Basis nachwachsender pflanzlicher Rohstoffe erhältlich sein.

### Gegenstand der Erfindung

Überraschenderweise wurde gefunden, dass Ester von 2-Hexyldecanol mit kurzkettigen Fettsäuren sich eignen für kosmetische und/oder pharmazeutische Zubereitungen, und in diesen Zubereitungen insbesondere als sensorisch besonders vorteilhafte Ölkörper eingesetzt werden können.

Gegenstand der Erfindung sind somit Ester von 2-Hexyldecanol mit Fettsäuren der allgemeinen Formel

(I) R₁-COOH,

wobei R₁ einen Alkylrest mit 1 bis 6 C Atomen darstellt.

Als Alkoholkomponenten des Esters wird 2-Hexyldecanol eingesetzt. 2-Hexyldecanol (synonym 2-Hexyl-1-decanol oder 2-Hexyldecylalcohol) ist kommerziell erhältlich, so z.B. unter dem Handelsnamen Eutanol®G 16 (Fa. Cognis Deutschland GmbH & Co. KG) oder als Exxal®16 (Exxon Chemical Company). 2-Hexyldecanol ist ein so genannter Guerbet-Alkohol, der durch Selbstkondensation von (primären) Alkoholen unter dem Einfluss von Natrium oder Kupfer bei erhöhter Temperatur und Druck erhältlich ist.

Als Fettsäurekomponente des Esters werden Fettsäuren der allgemeinen Formel (I)

R₁-COOH eingesetzt

wobei R₁ Alkylrest mit 1 bis 6 C Atomen darstellt, R₁ kann einen linearen oder verzweigten, gesättigten oder ungesättigten, aromatischen oder aliphatischen Alkylrest darstellen.

3-enyl, But-1-enyl, n-Pentyl, 1-Methylbutyl-, 2-Methylbutyl-, 3-Methylbutyl, 1-Ethylpropyl, 1,1-Dimethylpropyl, 1,2-Dimethylpropyl, 2,2-Dimethylpropyl, 1-Pentenyl-, 2-Pentenyl-, 3-Pentenyl-, 4-Pentenyl, Hexyl-, 1-Methylpentyl-, 2-Methylpentyl, 3-Methylpentyl, 4-Methylpentyl, 1-Ethylbutyl-, 2-Ethylbutyl-, 3-Ethylbutyl-,1-Hexenyl, 2-Hexenyl, 3-Hexenyl, 4-Hexenyl-, 5-Hexenyl-, Benzyl-.

In einer bevorzugten Ausführungsform der Erfindung wird eine Fettsäure der allgemeinen Formel (I) eingesetzt, in der R₁ einen linearen oder verzweigten, gesättigten Alkylrest mit 1 bis 6 C Atomen darstellt, besonders bevorzugt einen linearen, gesättigten Alkylrest mit 1 bis 6 C Atomen.

Besonders geeignete Ester im Sinne der Erfindung sind
2-Hexyldecyl-essigsäureester (R₁ = CH₃),
2-Hexyldecyl-propansäureester (R₁ = C₂H₅),
2-Hexyldecyl-butansäureester (R₁ = C₃H₇),
2-Hexyldecyl-pentansäureester (R₁ = C₄H₉),
2-Hexyldecyl-hexansäureester (R₁ = C₅H₁₁),
2-Hexyldecyl-heptansäureester (R₁ = C₆H₁₃),

### Herstellung

Die Herstellung der erfindungsgemäßen Ester erfolgt nach dem Fachmann bekannten Methoden der Veresterung. So kann z.B. die Fettsäure zusammen mit dem Hexyldecanol in Gegenwart eines Katalysators verestert werden.

Sowohl die Alkoholkomponenten als auch die Fettsäurekomponente können aus pflanzlichen Rohstoffen, wie beispielsweise Palmkern- oder Kokosöl gewonnen werden. Somit sind die erfindungsgemäßen Ester vollständig auf Basis nachwachsender Rohstoffe erhältlich.

### Verwendung in kosmetischen und/oder pharmazeutischen Zubereitungen

Überraschenderweise wurde festgestellt, dass sich die erfindungsgemäßen Ester besonders eignen zur Herstellung von kosmetischen Zubereitungen, sie eignen sich insbesondere als Ölkörper/Emollients und/oder Konsistenzgeber in kosmetischen Zubereitungen. Die erfindungsgemäßen Ester eignen sich weiterhin zur Herstellung von pharmazeutischen Zubereitungen, wobei die erfindungsgemäßen als technische Hilfsstoffe, wie z.B. Ölkörper eingesetzt werden. Die erfindungsgemäßen Ester können zur Herstellung von kosmetischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Bevorzugt ist die Verwendung der erfindungsgemäßen Ester als Ölkörper.

Die erfindungsgemäßen Ester können in kosmetischen Formulierungen als sog. ,light emollients' verwenden werden, um spezielle Eigenschaften, wie z.B. Spreitverhalten oder Flüchtigkeit einzustellen. Die erfindungsgemäßen Ester ermöglichen weiterhin viskositätsstabile kosmetische Formulierungen herzustellen.

Die erfindungsgemäßen Ester können sowohl einzeln als auch in beliebigen Mischungen untereinander eingesetzt werden.

### Beispiele

### Herstellungsbeispiel:

1 mol Hexansäure (116 g) Hexansäure, 1,1 mol (297 g) 2-Hexyldecanol (Guerbitol ®16) 16 sowie 0,22g Fascat® 2001 (Zinnoxalat) wurden zusammen gegeben und für 3h auf 240°C am Wasserabscheider erhitzt, Anschliessend wurde das Produkt über eine 30 cm Kolonne destilliert (153-168 °C bei 0,8 mbar). Das Produkt fällt als farbloses und geruchloses Öl an.

## Patentansprüche

1. Ester von 2-Hexyldecanol mit Fettsäuren der allgemeinen Formel (I)
R₁-COOH,
wobei R₁ Alkylrest mit 1 bis 6 C Atomen darstellt.

2. Ester nach Anspruch 1 ausgewählt aus der Gruppe bestehend aus 2-Hexyldecyl-essigsäureester, 2-Hexyldecyl-propansäureester, 2-Hexyldecyl-butansäureester,2-Hexyldecyl-pentansäureester, 2-Hexyldecyl-hexansäureester, und 2-Hexyldecyl-heptansäureester.

3. Verwendung der Ester nach einem der vorgenannten Ansprüche in und/oder zur Herstellung von kosmetischen und/oder pharmazeutischen Zubereitungen.

4. Verwendung nach Anspruch 3 als Ölkörper.
